# EUROPEAN PATENT APPLICATION

(11) **EP 4 082 458 A1**
(43) Date of publication of application: **02.11.2022**
(21) Application number: 21171669.1
(22) Date of filing: 30.04.2021
(51) Int. Cl.: A61B 17/86, A61B 17/70

(54) **BONE SCREW**

(71) Applicant: Biedermann Technologies GmbH & Co. KG, 78166 Donaueschingen (DE)
(72) Inventor: BIEDERMANN, Timo, 78647 Trossingen (DE); BIEDERMANN, Lutz, 78048 VS-Villingen (DE); DANNECKER, Berthold, 78112 St. Georgen (DE)
(74) Representative: Prüfer & Partner mbB Patentanwälte · Rechtsanwälte

(57) **Abstract**

A bone screw comprising a shank (2), the shank (2) having a screw axis (S) defining a longitudinal direction and further having a tip (2a) configured to be inserted first into bone, the shank (2) comprising a core (6) and a thread (7) winding in a helix with a plurality of turns around at least a portion of the core (6), the thread (7) being configured to engage bone, wherein the thread (7) comprises an upper flank (7a) facing away from the tip (2a) and a lower flank (7b) flank facing towards the tip (2a), wherein in a first section (Pi) of the thread (7) that is closer to the tip (2a), the upper flank and the lower flank (7b) form a first angle (α) with each other and in a second section (P₂) of the thread that is farther away from the tip (2a) than the first section (P₁), the upper flank (7a) and the lower flank (7b) form a second angle (β) with each other that is greater than the first angle (α).

## Description

The invention relates to a bone screw. In particular the invention relates to a bone screw that may be used in orthopedic surgery, more particular in spinal surgery.

In orthopedic surgery, bone screws that comprise a thread on their outer surface that is configured to engage bone are widely used for anchoring stabilization devices such as rods and/or plates in the bone. Various developments of bone screws have been made to fulfil specific requirements, such as a self-cutting ability and/or an increased resistance resistance to pull-out forces.

US 5,120,171, for example, describes a bone screw having a helical thread that gradually increases in thickness from the tip to the head of the screw. The thin thread at the tip of the screw can be inserted into a bone with minimal tearing or cracking of the bone. The thicker threads ascending from the tip to the head of the screw displaces bone against the superior (top) thread surfaces. This displacement of bone increases the screw's resistance to being pulled-out of the bone.

It is an object to provide an improved bone screw and a method of manufacturing the bone screw which is simple and effective in use.

The object is solved by a bone screw according to claim 1 and by a method according to claim 15. Further developments are given in the dependent claims. The further developments of the bone screw can also be used as further developments of the method.

The bone screw comprises a shank configured to be anchored in bone, wherein the shank has a screw axis defining a longitudinal direction and further has a tip configured to be inserted first into bone. The shank comprises a core and a thread winding in a helix with a plurality of turns around at least a portion of the core, the thread being configured to engage bone and comprises an upper flank facing away from the tip and a lower flank facing towards the tip. In a first section of the thread that is closer to the tip, the upper flank and the lower flank form a first angle with each other and in a second section of the thread that is farther away from the tip than the first section, the upper flank and the lower flank form a second angle with each other that is greater than the first angle.

Due to the smaller thread angle in the first section at or close to the tip of the bone screw, the thread is sharpened in the first section which allows to engage the cortical bone more easily. This may facilitate the insertion into bone. The necessary insertion torque may be reduced compared to a thread which has the greater angle between the upper flank and the lower flank also in the region at or adjacent to the tip.

Moreover, due to the greater thread angle in the second section that is closer to the neck of the bone screw, the resistance against pull-out forces, which tend to pull-out the bone screw once it has been inserted, may be increased.

A method for manufacturing the bone screw includes a step of providing a bone screw with a bone thread having a constant thread angle along a substantial part of the length of the thread and a step in which in a region that extends from a position at or close to the tip along a portion of the shank a thread with a smaller thread angle is cut, respectively milled. Hence, the method is easy to perform and cost saving.

Further features and advantages of the invention will become apparent from the description of embodiments by means of the accompanying drawings.

In the drawings:
- Fig. 1:: shows a perspective exploded view of an embodiment of a polyaxial bone anchoring device including a first embodiment of a bone screw.
- Fig. 2:: shows a perspective view of the polyaxial bone anchoring device of Fig. 1 in an assembled state.
- Fig. 3:: shows a cross-sectional view of the polyaxial bone anchoring device of Figs. 1 and 2, the cross-section being taken in a plane including the screw axis of the bone screw and extending through a center of the receiving part perpendicular to a rod channel.
- Fig. 4:: shows an enlarged perspective view of a portion of the shank of the bone screw of Figs. 1 to 3.
- Fig. 5:: shows a side view of the bone screw shown in Figs. 1 to 4.
- Fig. 6:: shows a side view of the bone screw of Fig. 5 rotated by 90°.
- Fig. 7:: shows a cross-sectional view of the bone screw of Fig. 6, the cross-section being taken in a plane extending through the center of the bone screw in the longitudinal direction.
- Fig. 8:: shows a side view of a second embodiment of the bone screw.
- Fig. 9:: shows a side view of the bone screw of Fig. 8 rotated by 90°.
- Fig. 10:: shows side view of a third embodiment of the bone screw.
- Fig. 11:: shows a side view of the bone screw of Fig. 10 rotated by 90°.

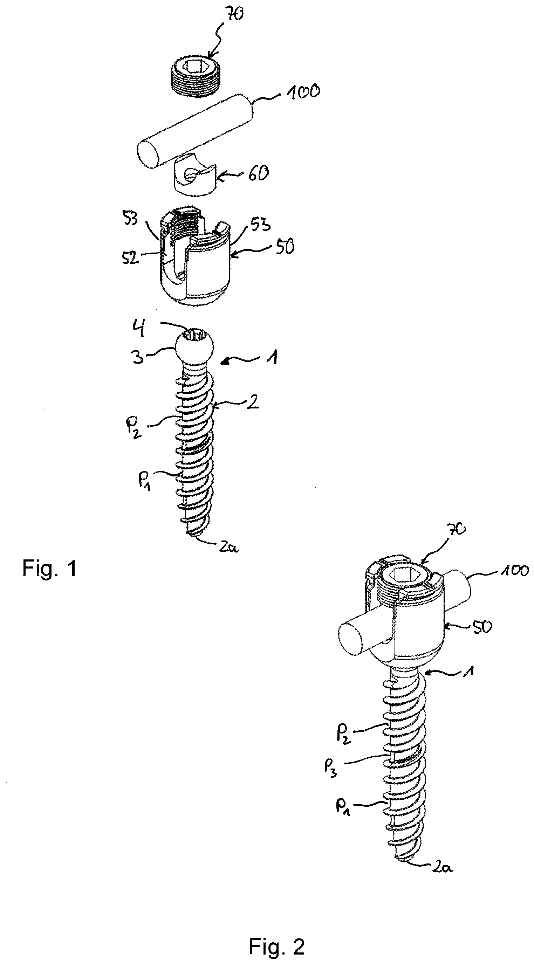

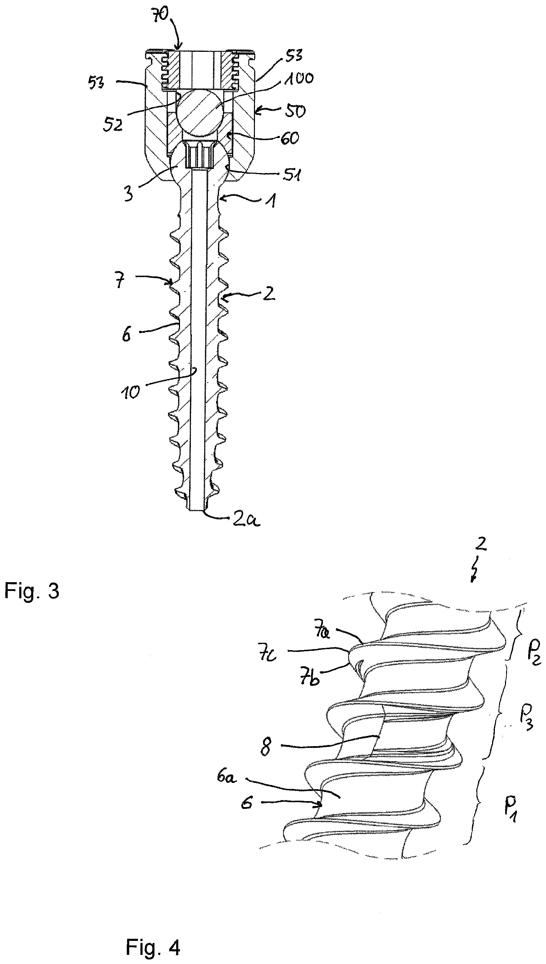

Referring to Figs. 1 to 7, a first embodiment of the bone screw in connection with a polyaxial bone anchoring device is described. The bone screw 1 comprises a shank 2 with a tip 2a at one free end that is configured to enter the bone first. The tip may be blunt or sharp or may have any suitable shape. At the end of the shank 2 opposite to the tip a head 3 may be formed that may have a spherical outer surface portion and a free end surface 3a. More specifically, the head 3 may have the shape of a spherical segment having a substantially flat end surface 3a. Further, an engagement recess 4 may be provided in the free end surface 3a of the head 3 for engagement with a drive tool. Adjacent to the head 3 a reduced diameter neck section 5 may be formed. The bone screw may be part of a polyaxial bone anchoring device. Such a polyaxial bone anchoring device typically includes the bone screw 1, a receiving part 50 and a pressure member 60. The receiving part 50 comprises an accommodation space having a seat 51 for the head 3 that enables the head 3 to pivot in the seat similar to a ball and a socket joint so that the shank 2 can assume various angular positions. Furthermore, the receiving part 50 may have a recess 52 for receiving a spinal rod 100 and a locking member 70, for example in the form of a screw member cooperating with internally threaded legs 53 of the receiving part for locking the head 3 and the rod 100 in the receiving part 50. The head 3 can be locked in a particular angular position by tightening the locking member 70 which in turn exerts pressure via the rod and the pressure member 60 on the head 3. There may be various designs of such a polyaxial bone anchoring device and the present disclosure is not limited to the design shown in the Figures.

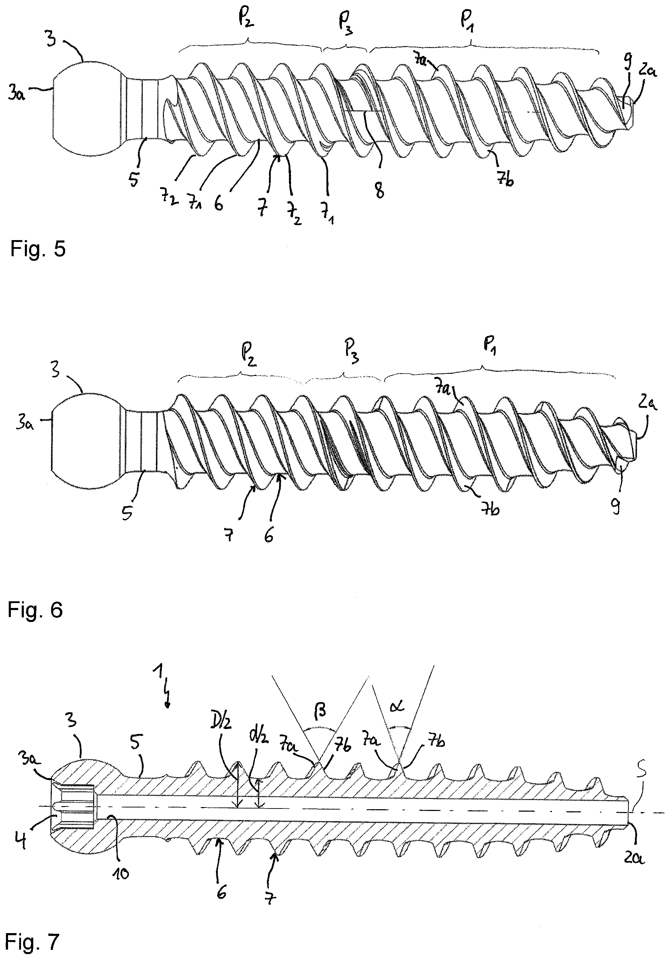

The shank 2 of the bone screw 1 comprises a core 6 and a thread 7 winding in a helix around the core 6 in a plurality of turns. In the specific embodiment, the thread 7 is a double start thread, i.e. two helices 7₁, 72 each having a separate thread entry are winding around the core 6. By the thread 7, a longitudinal axis or screw axis S is defined which also defines the axial direction of the bone screw 1. An outer diameter of the core or core diameter d, wherein d/2 is indicated in Fig. 7, may be constant over at least most of the length of the core 6. In a region adjacent to the tip 2a, the core diameter d may, however, decrease to form a tapered tip 2a. In the embodiment, the thread 7 extends from the tip 2a up to the neck section 5. In greater detail, the thread 7 comprises a first or lower flank 7a facing towards the tip 2a, and a second or upper flank 7b facing towards the head 3. The upper flank 7a and the lower flank 7b join in a crest 7c at the outermost portion of the thread turn. The shape of the crest may be sharp or rounded or flat. A cross-section of the thread 7 in a plane including the screw axis S may be substantially triangular, in particular with equilateral sides, more specifically the cross-section may be that of a V-thread. Moreover, a thread pitch, which is the distance in the axial direction from one crest 7c to the neighboring crest 7c , may be constant along substantially the entire length of the thread. In the case of the bone thread, the thread pitch may be such that there is a gap 6a between the thread turns on the core 6. The outer diameter D of the thread which is twice the distance from the screw axis to the crest 7c, shown as D/2 in Fig. 7, may be constant along substantially the length of the thread. However, the outer diameter D may decrease in a region towards the tip 2a and in a run-out region towards the neck 5. As the thread 7 in this embodiment is a double start thread, the lead, i.e. the distance the screw travels with one revolution, is two times the pitch.

As best seen in Figs. 5 to 7, in a first section P₁ of the thread 7 that starts at or close to the tip 2a, the upper flank 7a and the lower flank 7b of the thread 7 form a first angle α with each other. In a second section P₂ which is adjacent to or close to the neck section 5, the upper flank 7a and the lower flank 7b of the thread form a second angle β with each other. The first angle α is smaller than the second angle β. Since the first angle is smaller than the second angle, the thickness of the thread in the axial direction is smaller in the first section than in the second section. The second angle β is in the embodiment 60° which is the angle of a 60° V-thread. The first angle α is in the embodiment 40°. It should be noted that values for the first and the second angle for all embodiments cover at least manufacturing and/or measuring tolerances. The thread angle is measured between two flanks, upper flank 7a and lower flank 7b that are facing each other, in a plane including the screw axis S. In Fig. 7, the thread angle is shown as the angle formed by the two intersecting planes extending through the upper flank 7a and the lower flank 7b, respectively, which corresponds to the angle measured between two opposing flanks.

Preferably, the ratio between the first angle α and the second angle β is in the range between about 0.1 to 0.9, more preferably between 0.2 and 0.8, still more preferably between 0.4 and 0.7.

Between the first section P₁ and the second section P₂ a transition section P₃ may be formed in which the thread angle changes between the first angle α and the second angle β. The transition section P₃ may be defined by a run-out zone in which the tool that produces the thread with the first angle α runs-out. Hence, along a portion of one turn of the thread in the transition zone P₃, the upper flank 7a and the lower flank 7b each have a portion with both, the first thread angle α and also the second thread angle β. The transition section P₃ starts in the helical direction at a position 8 which corresponds to the start of the removal of a tool that generates the thread with the first angle α in the first section P₁. It ends at an axial position in the direction towards the neck section 5 in which the upper and lower flanks 7a, 7b are free from portions with the first thread angle α.

For facilitating insertion in the bone, the thread may have a cutting feature 9 at or close to the tip 2a. The cutting feature may be a recess in the thread that sharpens the thread preferably in the first thread turn adjacent to the tip 2a. Moreover, the shank 2 may be conical in a region adjacent to the tip 2a. This can be achieved by either reducing the core diameter and/or the outer diameter of the thread 7.

Lastly, a channel 10 may extend from the end surface of the head 3 entirely through the shank 2 up to the tip 2a. This may be useful for guiding a guidewire therethrough or for injecting substances. The channel 10 is preferably coaxial with the shank axis S and has a circular cross-section. An inner diameter of the channel may be substantially constant over the length. However, the inner diameter may also vary along the length and/or the cross-section may the other than circular. In a further embodiment (not shown) the shank may be fenestrated, i.e. it may have one or a plurality of openings that connect the channel 10 with the outside of the bone anchor.

A method of manufacturing the bone screw 1 comprises at least a first step of providing a bone screw with a thread 7 along a length of the shank 2 that includes a portion at or close to the tip 2a, wherein the thread 7 comprises a thread angle that is constant along the length of the thread 7 and that corresponds to the second angle β in the second section P₂. The thread may be a V-thread and the second angle β may be 60°. Then, in a second step, a first section P₁ at or close to the tip 2a is generated that has the first thread angle α. The first angle α is smaller than the second angle β and may be 40°. Preferably, the first section P₁ is generated by machining the shank 2 with a tool, more preferably the thread is cut or milled with a thread cutter that starts at the tip and runs out from the position 8 which indicates the beginning of the removal of the tool. The remaining section P₂ adjacent to the first section, P₁ more specifically adjacent to the transition section P₃ has the second angle β.

Generally, with the method, various other embodiments can be generated in which a first section is different from the second section with regard to the thread angle and/or with regard to the thread form. For example, the first section which is generated in the second step may be different from the second section also with regard to the threadform. Methods in which material is removed from a bone screw may be preferred as they are simple and cost saving. However, it can be also envisaged to generate the entire bone screw in one step using an additive manufacturing method, for example an additive layer manufacturing method such as laser or electron beam melting in which the bone screw is built up in a layer-wise manner based on CAD data of the final bone screw.

The bone anchor 1 may be made of any bio-compatible material, preferably however of titanium or stainless steel or of any other bio-compatible metal or metal alloy or plastic material. As a bio-compatible alloy, a NiTi alloy, for example Nitinol, may be used. Other materials can also be magnesium or magnesium alloys. Bio-compatible plastic materials for use may be, for example, polyether ether ketone (PEEK) or poly-L-lactide acid (PLLA).

In the clinical use, the bone screw is inserted into bone, for example in the pedicle of a vertebra. In one method of use, the bone screw is first inserted into bone and another device, such as the receiving part of a polyaxial bone anchoring device, which is exemplary shown in Figs. 1 to 3, or a bone plate is mounted onto the head 3 of the inserted bone screw. In another method of use, the bone screw is pre-assembled with the other device and thereafter inserted into bone. When the bone screw is inserted, the first section P₁ of the shank 2 with the smaller first thread angle α facilitates the engagement of the thread 7 with the bone. In addition, the insertion torque is reduced. Thus, the bone screw can easily engage and traverse the cortical bone. Upon further insertion, the second section P₂ engages the bone until the bone screw is fully inserted. Due to the second section P₂ having the greater thread angle β, the resistance against pull-out is similar to that of a usual bone screw with a 60° V-thread extending along its entire length. It may also be increased compared to a screw with a constant 60° V-thread as the bone is condensed around the second section P₂ during insertion.

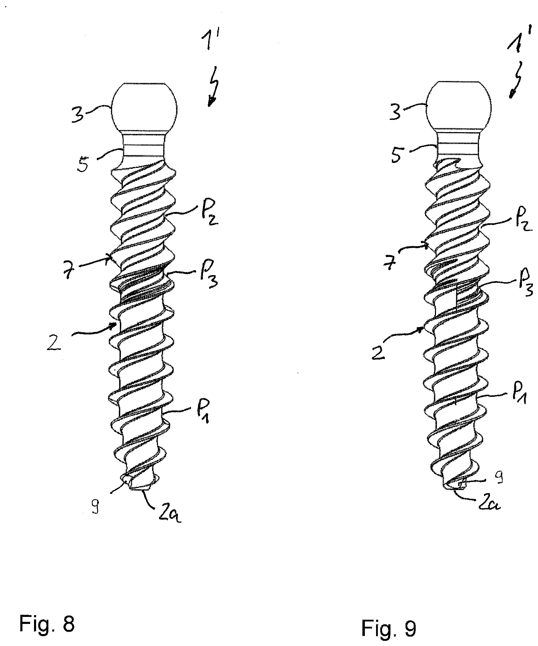

In Figs. 8 and 9, a second embodiment of the bone screw is shown. The bone screw 1' differs from the bone screw only by the thread angles of the first and second sections. The second thread angle β' is in this case about 80° and the first thread angle α' is about 40°. The second angle β' in this embodiment is about twice the first angle α'. Such a difference may be useful in the case of known or expected higher pull-out forces that may act on the bone screw.

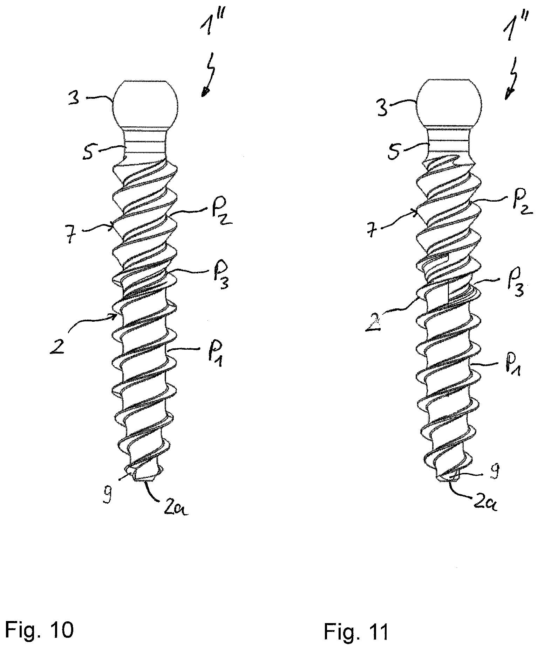

In Figs. 10 and 11 a third embodiment of the bone screw is shown. The bone screw 1" differs from the bone screw of the previous embodiments only by the thread angle. The second angle β" in the second section P₂ is about 50° and the first angle α" of the first section P₁ is about 30°. This configuration may be useful, for example, in the case of healthy bone for which a reduced insertion torque facilitates the insertion while a risk of pull-out may be not overly high.

In the embodiments shown, the thread 7 is a double start thread. In a still further embodiment, the thread may be a single start thread or a triple or a multiple start thread. Also in such an embodiment, the bone screw has first and the second sections with the corresponding thread angles as in the previous embodiments.

While the thread 7 is shown as a V-thread, other known threadforms can also be used as long as they fulfil the requirement to engage bone. For example, trapezoidal threads, buttress threads, and other threads may be used.

The specific thread shape, the pitch, the number of threads, etc. are parameters that may depend on the type of bone which the anchor is to be inserted and on the purpose of the bone anchor.

While only two sections with different thread angles are described, it may be conceivable that more than two sections with different thread angles are formed. For example, a first section with a first thread angle could be formed adjacent to the tip, followed by a second section with a greater thread angle than that of the first section and further followed by a third section with a greater thread angle than that of the second section in the direction towards the neck.

It shall be noted that while the pitch and the cross-section of the thread 7 remains substantially the same in the embodiments shown, there may be other embodiments where the pitch is different in the two sections having the different thread angles.

Other modifications may be possible. The shank may also have thread free portions, i.e. the thread 7 may be present only in a portion or portions of the shank. The tip 2a needs not to be conical but may be formed by the end surface of a cylindrical shank or can be just any other end portion of the shank that is configured to be inserted into bone first. The bone screw may be headless or the head may have another shape. Various self-cutting features may also be provided, in particular in the first section and/or the thread may be completely self-cutting.

For the polyaxial bone anchoring device all kinds of polyaxial bone anchoring devices may be used.

## Claims

1. Bone screw comprising
a shank (2) configured to be anchored in bone, the shank (2) having a screw axis (S) defining a longitudinal direction and further having a tip (2a) configured to be inserted first into bone, the shank (2) comprising
a core (6) and
a thread (7) winding in a helix with a plurality of turns around at least a portion of the core (6), the thread (7) being configured to engage bone,
wherein the thread (7) comprises an upper flank (7a) facing away from the tip (2a) and a lower flank (7b) flank facing towards the tip (2a), wherein in a first section (P₁) of the thread (7) that is closer to the tip (2a), the upper flank and the lower flank (7b) form a first angle (α) with each other and in a second section (P₂) of the thread that is farther away from the tip (2a) than the first section (P₁), the upper flank (7a) and the lower flank (7b) form a second angle (β) with each other that is greater than the first angle (α).

2. The bone screw of claim 1, wherein the first section (P₁) and the second section (P₂) are adjacent to each other in the longitudinal direction.

3. The bone screw of claim 1 or 2, wherein a pitch of the thread (7) is the same in the first section (P₁) and the second section (P₂).

4. The bone screw of one of claims 1 to 3, wherein an outer diameter (d) of the core (6) is constant along at least a part of the first section (P₁) and the second section (P₂), preferably including a region where the first and the second section merge in the axial direction.

5. The bone screw of one of claims 1 to 4, wherein an outer diameter (D) of the thread (7) is constant along at least a part of the first section (P₁) and the second section (P₂), preferably including a region where the first section and the second section merge in the axial direction.

6. The bone screw of one of claims 1 to 5, wherein the first section (P₁) comprises at least two turns, preferably wherein the first section extends along at least between about a quarter to about three quarters of the length of the shank (2) in the longitudinal direction.

7. The bone screw of one of claims 1 to 6, wherein the second section (P₂) comprises at least two turns, preferably wherein the second section (P₂) has about the same length as the first section (P₁) or is shorter in the longitudinal direction than the first section (P₁).

8. The bone screw of one of claims 1 to 7, wherein a ratio between the first angle (α) and the second angle (β) is in the range between about 0.1 to 0.9, more preferably between 0.2 and 0.8, still more preferably between 0.4 and 0.7.

9. The bone screw of one of claims 1 to 8, wherein the first angle (α) is between about 10° to about 60° and the second angle (β) is between about 20° to about 90°, preferably wherein the first angle (α) is between about 20° to about 50° and the second angle (β) is between about 30° to about 80°.

10. The bone screw of one of claims 1 to 9, wherein the first section (P₁) is located adjacent to the tip (2a).

11. The bone screw of one of claims 1 to 10, wherein the cross-section of the thread (7) is symmetrical with respect to a plane perpendicular the screw axis (S) axis.

12. The bone screw of one of claims 1 to 11, further comprising a transition zone (P₃) between the first section (P₁) and the second section (P₂) in which the upper flank (7a) and/or the lower flank (7b) have run-out portions of the first angle (α) in the direction of the helix,

13. The bone screw of one of claims 1 to 12, further comprising a head (3) at the end opposite to the tip (2a), preferably with a spherical outer surface portion.

14. A polyaxial bone anchoring device comprising a bone screw of claim 13, and a receiving part (50) for coupling the bone screw (1) to a rod (100), the receiving part comprising a seat (51) for pivotably receiving the head (3) and a recess (52) for receiving the rod (100).

15. A method of manufacturing a bone screw, comprising the steps of
providing an at least partially threaded shank (2) which comprises a thread (7) configured to engage bone and a tip (2a), wherein the thread (7) comprises an upper flank (7a) facing away from the tip (2a) and a lower flank facing towards the tip (2a), and wherein the upper flank (7a) and the lower flank (7b) form a second angle (β) with each other ;
removing material from the upper flank (7a) and the lower flank (7b) along a portion of the thread (7) such that a first section (P₁) is produced in which the upper flank (7a) and the lower flank (7b) form a first angle (α) with each other that is smaller than the second angle (β), preferably wherein the first section is closer to the tip (2a) than the second section.

## Amended claims

### Amended claims in accordance with Rule 137(2) EPC.

1. Bone screw comprising
a shank (2) configured to be anchored in bone, the shank (2) having a screw axis (S) defining a longitudinal direction and further having a tip (2a) configured to be inserted first into bone, the shank (2) comprising
a core (6) and
a thread (7) winding in a helix with a plurality of turns around at least a portion of the core (6) , the thread (7) being configured to engage bone,
wherein the thread (7) comprises an upper flank (7a) facing away from the tip (2a) and a lower flank (7b) flank facing towards the tip (2a), wherein in a first section (P₁) of the thread (7) that is closer to the tip (2a), the upper flank and the lower flank (7b) form a first angle (α) with each other and in a second section (P₂) of the thread that is farther away from the tip (2a) than the first section (Pi), the upper flank (7a) and the lower flank (7b) form a second angle (β) with each other that is greater than the first angle (α) and
wherein an outer diameter (d) of the core (6) is constant along at least a part of the first section (P₁) and the second section (P₂).

2. The bone screw of claim 1, wherein a pitch of the thread (7) is the same in the first section (P₁) and the second section (P₂).

3. The bone screw of claim 1 or 2, wherein the part of the first section (P₁) and the second section (P₂) where the outer diameter (d) of the core (6) is constant includes a region where the first and the second section merge in the axial direction.

4. The bone screw of one of claims 1 to 3, wherein an outer diameter (D) of the thread (7) is constant along at least a part of the first section (P₁) and the second section (P₂), preferably including a region where the first section and the second section merge in the axial direction.

5. The bone screw of one of claims 1 to 4, wherein the first section (P₁) comprises at least two turns, preferably wherein the first section extends along at least between about a quarter to about three quarters of the length of the shank (2) in the longitudinal direction.

6. The bone screw of one of claims 1 to 5, wherein the second section (P₂) comprises at least two turns, preferably wherein the second section (P₂) has about the same length as the first section (P₁) or is shorter in the longitudinal direction than the first section (P₁).

7. The bone screw of one of claims 1 to 6, wherein a ratio between the first angle (α) and the second angle (β) is in the range between about 0.1 to 0.9, more preferably between 0.2 and 0.8, still more preferably between 0.4 and 0.7.

8. The bone screw of one of claims 1 to 7, wherein the first angle (α) is between about 10° to about 60° and the second angle (β) is between about 20° to about 90°, preferably wherein the first angle (α) is between about 20° to about 50° and the second angle (β) is between about 30° to about 80°.

9. The bone screw of one of claims 1 to 8, wherein the first section (P₁) is located adjacent to the tip (2a).

10. The bone screw of one of claims 1 to 9, wherein the cross-section of the thread (7) is symmetrical with respect to a plane perpendicular the screw axis (S) axis.

11. The bone screw of one of claims 1 to 10, wherein the first section (P₁) and the second section (P₂) are adjacent to each other in the longitudinal direction.

12. The bone screw of one of claims 1 to 10, further comprising a transition zone (P₃) between the first section (P₁) and the second section (P₂) in which the upper flank (7a) and/or the lower flank (7b) have run-out portions of the first angle (α) in the direction of the helix.

13. The bone screw of one of claims 1 to 12, further comprising a head (3) at the end opposite to the tip (2a), preferably with a spherical outer surface portion.

14. A polyaxial bone anchoring device comprising a bone screw of claim 13, and a receiving part (50) for coupling the bone screw (1) to a rod (100), the receiving part comprising a seat (51) for pivotably receiving the head (3) and a recess (52) for receiving the rod (100).

15. A method of manufacturing a bone screw, comprising the steps of
providing an at least partially threaded shank (2) which comprises a thread (7) configured to engage bone and a tip (2a), wherein the thread (7) comprises an upper flank (7a) facing away from the tip (2a) and a lower flank facing towards the tip (2a), and wherein the upper flank (7a) and the lower flank (7b) form a second angle (β) with each other;
removing material from the upper flank (7a) and the lower flank (7b) along a portion of the thread (7) such that a first section (P₁) is produced in which the upper flank (7a) and the lower flank (7b) form a first angle (α) with each other that is smaller than the second angle (β), preferably wherein the first section is closer to the tip (2a) than the second section.
